# EUROPÄISCHE PATENTSCHRIFT

(11) **EP 3 907 217 B1**
(45) Veröffentlichungstag und Bekanntmachung des Hinweises auf die Patenterteilung: **08.06.2022**
(21) Anmeldenummer: 21171186.6
(22) Anmeldetag: 29.04.2021
(51) Int. Cl.: C07D 211/74

(54) **VERBESSERTES VERFAHREN ZUR HERSTELLUNG VON TRIACETONAMIN**
IMPROVED METHOD FOR THE PREPARATION OF TRIACETONAMINE
PROCÉDÉ AMÉLIORÉ DE PRODUCTION DE TRIACÉTONAMINE

(30) Priorität: 07.05.2020 EP 20173463
(43) Veröffentlichungstag der Anmeldung: 10.11.2021
(73) Patentinhaber: Evonik Operations GmbH, 45128 Essen (DE)
(72) Erfinder: Minke, Katharina, 45128 Essen (DE); Graskamp, Holger, 45768 Marl (DE)
(74) Vertreter: Evonik Patent Association

(56) Entgegenhaltungen:
- CN-A- 108 383 704
- DE-A1- 2 807 172

## Beschreibung

Die vorliegende Erfindung betrifft ein verbessertes Verfahren zur Herstellung von Triacetonamin. Dabei erfolgt ein Absorptionsschritt, in welchem im Rohprodukt enthaltenes Aceton destillativ von Triacetonamin abgetrennt und dann gasförmig im Gegenstrom in frisches, flüssiges Aceton absorbiert wird. Der so erhaltene Acetonstrom wird dann weiter zu Triacetonamin umgesetzt. Dieses Verfahren erlaubt eine energieeffizientere Wiederverwertung der bei der Synthese von Triacetonamin eingesetzten und nicht reagierten Edukte und senkt somit insgesamt sowohl den Einsatz an Edukten als auch den Aufwand an Energie.

### Hintergrund der Erfindung

Triacetonamin (2,2,6,6-Tetramethyl-4-piperidinon; CAS-Nummer: 826-36-8; im Folgenden "TAA") ist ein wichtiges chemisches Intermediat, welches zur Synthese zahlreicher Folgeprodukte wie beispielsweise Lichtstabilisatoren *(hindered amine light stabilizers;* [HALS]), Oxidationsmitteln und Polymerisationsmoderatoren (z. B. Nitroxyl-Radikale) eingesetzt wird.

Die Herstellung von Triacetonamin aus Aceton und Ammoniak ist in Form verschiedener Verfahren dokumentiert. Dabei gliedern sich die Herstellungsverfahren in die direkte (einschrittige) Synthese von TAA aus den Edukten, beispielsweise beschrieben in DE 24 29 937 A1, US 4,536,581 A, JPS54-88275 A oder in Zeitschrift für Naturforschung 1976, 328 - 337 und 338 - 345, sowie die indirekte (zweistufige) Synthese über Acetonin (2,2,4,4,6-Pentamethyl-1,2,5,6-tetrahydro-pyrimidin), beispielsweise beschrieben in DE 24 29 935 A1 oder DE 24 29 936 A1, oder über Phoron (2,6-Dimethyl-2,5-heptadien-4-on), z. B. beschrieben in DE 2 352 127 A1. Bei der zweistufigen TAA-Synthese über Acetonin wird dies zunächst ausgehend von Aceton und Ammoniak gebildet, und reagiert dann in einem anschließenden Schritt unter Abspaltung von einem Äquivalent Ammoniak weiter zu TAA. Im Falle des Syntheseverfahrens über Acetonin werden jedoch immer beide Spezies (TAA und Acetonin) gleichzeitig gebildet, die Acetoninbildung ist allerdings kinetisch gegenüber der TAA-Bildung stark bevorzugt. In der "einstufigen" TAA-Synthese wird Acetonin lediglich nicht isoliert.

Die Herstellung von TAA ist grundsätzlich sowohl homogen katalysiert (meist durch Ammoniumsalze) als auch heterogen katalysiert (z. B. an sauren lonentauschern) möglich.

Die meisten Schriften aus dem Stand der Technik beziehen sich auf homogen katalysierte Reaktionen. Am häufigsten werden dabei Calciumchlorid (z.B. in Chemical Industries 2003, 89, 559-564; Zeitschrift für Naturforschung 1976, 328 - 337 und 338 - 345), Ammoniumchlorid (z. B. in JP 2003-206277 A; JP 2001-31651 A; JPH4-154762 A) und Hydrazinderivate (z. B. in JPS54-88275 A, JPS54-112873 A) genannt. Bei Einsatz dieser Katalysatoren treten allerdings Probleme auf. So hat etwa der Einsatz von Calciumchlorid den Nachteil, dass eine sehr langsame Reaktion erfolgt. Im Falle des Ammoniumchlorids ist die Reaktionsgeschwindigkeit höher, das eingesetzte Chlorid zeigt allerdings eine hohe Korrosivität gegenüber Stahl. Hydrazinderivate zeigen demgegenüber eine sehr hohe Toxizität.

Daneben wurden auch Reaktionen an heterogenen Katalysatoren beschrieben, wie zum Beispiel in der CN108 383 704 A, der EP 3 663 284 A1, der DE 28 07 172 A1 und der CN 103224465 A.

Im Allgemeinen wird TAA in einer Matrix hergestellt, bei der das Aceton in großem Überschuss vorliegt und sowohl als Reaktionspartner als auch als Lösungsmittel dient. Daher ergibt sich am Ende der Reaktion ein Rohprodukt, welches neben TAA einen großen Anteil an Aceton, nicht umgesetzten Ammoniak, durch die Kondensation gebildetes Wasser und bei homogenkatalytischen Verfahren den Katalysator enthält. Darüber hinaus sind weitere Nebenkomponenten enthalten, wie z. B. acyclische Kondensationsprodukte (z. B. Diacetonalkohol, Diacetonamin, Mesityloxid, Phoron etc.), cyclische Kondensationsprodukte [z. B. Acetonin, 2,2,4,6-Tetramethyl-2,3-dihydropyridin (im Folgenden "TMDH-Pyridin")] oder höhermolekulare Kondensationsprodukte ("Hochsieder").

Einige acyclische Additions- und Kondensationsprodukte (z. B. Diacetonalkohol [4-Hydroxy-4-methylpentan-2-on], Diacetonamin [4-Amino-4-methylpentan-2-on], Mesityloxid [4-Methylpent-3-en-2-on], Phoron etc.) können ihrerseits anstatt Aceton als Edukte mit Ammoniak zu TAA umgesetzt werden, was man sich beispielsweise in Verfahren mit einem prozessinternen Recyclingstrom zunutze macht.

Ein solcher prozessinterner Recyclingstrom ist in der DE 28 07 172 A1 beschrieben. Wie in den Beispielen 5a und 6a der DE 28 07 172 A1 beschrieben, werden dazu die einzelnen Komponenten des TAA-Rohprodukts destillativ aufgetrennt, und die reaktiven Komponenten Aceton und Mesityloxid dann wieder als Edukt eingesetzt.

Dazu wird das Rohprodukt aus der Synthese des TAA erhitzt, die einzelnen Komponenten abgetrennt, jeweils kondensiert und wieder rückgeführt. Dies bedeutet einen hohen Energieaufwand und führt auch zu Verlusten an Aceton, welche durch Zugabe frischen Acetons ins System ausgeglichen werden müssen.

Es bestand deshalb der Bedarf nach einem effizienten Verfahren zur Synthese von TAA, welches die vorgenannten Probleme nicht aufweist und insbesondere eine einfache, energieextensive und somit effiziente Wiederverwertung des bei der Umsetzung von TAA nicht reagierten Acetons ermöglicht.

Es wurde nun überraschend ein Verfahren gefunden, was diese Aufgabe löst.

### Detaillierte Beschreibung der Erfindung

Es wurde überraschend festgestellt, dass ein besonders effizientes Verfahren zur Herstellung von TAA dadurch ermöglicht wird, dass das überschüssige Aceton, welches aus dem Rohprodukt der TAA-Herstellung abgetrennt wird, zusammen mit frischem Aceton dem Verfahren erneut zugeführt wird. Dazu wird es aus dem Rohprodukt abgetrennt und gasförmig im Gegenstrom in das frische Aceton absorbiert. Dieses Verfahren vermeidet aufwendige Abtrennungsschritte, insbesondere die energieintensive Kondensation von Aceton, und erlaubt somit im Vergleich zu herkömmlichen Verfahren eine Wiederverwertung überschüssigen Acetons bei geringerem Energieaufwand.

Das erfindungsgemäße Verfahren ist demnach ein, bevorzugt kontinuierliches, Verfahren zur Herstellung von Triacetonamin umfassend die folgenden Schritte:
(a) Umsetzung eines Acetonstroms **S_{Ac1}** und eines Ammoniakstroms **S_{Am1}** in Gegenwart eines Katalysators **K1** zu einem Rohproduktstrom **S_{RP1}** umfassend Triacetonamin, Aceton,
(b) mindestens teilweise destillative Abtrennung von Aceton aus **S_{RP1}**, wodurch ein gasförmiger Strom **S_{Ac2}** umfassend Aceton erhalten wird,
(c) mindestens teilweise Absorption des gasförmigen Stroms **S_{Ac2}** im Gegenstrom in einen flüssigen Acetonstrom **S_{Ac3}**, wodurch ein flüssiger Acetonstrom **S_{Ac4}** erhalten wird,
(d) Wiederholung des Schrittes (a), wobei der Acetonstrom **S_{Ac4}** als Acetonstrom **S_{Ac1}** eingesetzt wird.

### 1. Schritt (a)

Im Schritt (a) des erfindungsgemäßen Verfahrens werden ein Acetonstrom **S_{Ac1}** und ein Ammoniakstroms **S_{Am1}** in Gegenwart eines Katalysators **K1** zu einem Rohproduktstrom **S_{RP1}** umfassend Triacetonamin, Aceton umgesetzt.

Dabei wird ein Rohproduktstrom **S_{RP1}** umfassend Triacetonamin, Aceton erhalten. Dieser umfasst neben dem gewünschten Produkt TAA somit auch nicht reagiertes Aceton. Gegebenenfalls umfasst der Rohproduktstrom **S_{RP1}** auch nicht reagierten Ammoniak sowie Nebenprodukte. Als Nebenprodukt kommt insbesondere Mesityloxid in Frage. Mesityloxid ist das einfachste Kondensationsprodukt, das stets zumindest in geringer Menge bei der Umsetzung von Ammoniak und Aceton zu TAA durch Aldolkondensation zweier Moleküle Aceton gebildet wird. Auch höhermolekulare Kondensationsprodukte des Acetons mit sich oder Ammoniak entstehen bei der Umsetzung im Schritt (a) und sind deshalb insbesondere vom Rohproduktstrom **S_{RP1}** umfasst. Dies sind vor allem Nebenprodukte ausgewählt aus der Gruppe bestehend aus Diacetonalkohol, Diacetonamin, Acetonin, Phoron, Isophoron, bevorzugt ausgewählt aus der Gruppe bestehend aus Diacetonalkohol, Diacetonamin, Acetonin, Isophoron.

Die Umsetzung in Schritt (a) wie auch das gesamte erfinderische Verfahren wird bevorzugt kontinuierlich durchgeführt.

Im kontinuierlichen Betrieb werden bevorzugt alle Chemikalien gleichzeitig bei der Reaktionstemperatur zudosiert. Bei der kontinuierlichen Reaktion kann jeder dem Fachmann bekannte Reaktor eingesetzt werden, z.B. ein kontinuierliches Strömungsrohr, ein kontinuierlicher Rührkessel, eine Rührkesselkaskade, sowie mögliche Kombinationen aus diesen einzelnen Elementen. Dabei wird bevorzugt eine Kombination von einem oder mehreren Reaktoren mit internem oder externem Kreislauf, gefolgt von einem Nachreaktor mit Strömungsrohrcharakteristik, eingesetzt.

Die Reaktionszeit in Schritt (a) im kontinuierlichen Betrieb ergibt sich durch die Gesamt-Verweilzeit der Reaktanden im Reaktor und liegt im Bereich von 1 bis 15 Stunden, bevorzugt im Bereich von 3 bis 9 Stunden, besonders bevorzugt im Bereich von 5 bis 7 Stunden.

Die *"volume space velocity"* für Ammoniak im kontinuierlichen Betrieb liegt in Schritt (a) insbesondere bei 0.01 bis 124.20 h⁻¹, bevorzugt 0.03 bis 5.25 h⁻¹, am bevorzugtesten bei 0.06 h⁻¹ (dies entspricht dem Volumen an Ammoniak, was pro Stunde und pro Volumen des Reaktors durch diesen Reaktor fließt, abgekürzt als "LHSV").

Die *"volume space velocity"* für Aceton im kontinuierlichen Betrieb liegt in Schritt (a) insbesondere bei 0.15 bis 1.33 h⁻¹, bevorzugt 0.66 bis 1.17 h⁻¹ (dies entspricht dem Volumen an Aceton, was pro Stunde und pro Volumen des Reaktors durch diesen Reaktor fließt).

Die Umsetzung in Schritt (a) kann in Gegenwart weiterer Lösungsmittel oder nur in Aceton, also ohne Zugabe weiterer Lösungsmittel, stattfinden. In den Fällen, in denen ein Lösungsmittel in Schritt (a) eingesetzt wird, können sämtliche Lösungsmittel eingesetzt werden, die die Reaktion nicht verhindern. Insbesondere sind die möglichen Lösungsmittel aliphatische Lösungsmittel, bevorzugt Pentan, Hexan, Heptan, Octan, Decan, Cyclohexan, Tetramethylsilan; aromatische Lösungsmittel, bevorzugt Benzol, Toluol, Xylol; Ether-Verbindungen, bevorzugt Diethylether, Dipropylether, Dibutylether, Methyl-*tert*-Butylether; halogenierte Lösungsmittel, bevorzugt Dichlormethan, Chloroform, Tetrachlormethan; Alkohole, bevorzugt Methanol, Ethanol, Propanol, Isopropanol, Butanol, *tert*-Butanol; Ester, bevorzugt Methylacetat, Ethylacetat, Propylacetat, Butylacetat. Besonders bevorzugt findet die Umsetzung in Schritt (a) in Aceton, ohne Zugabe weiterer Lösungsmittel, statt.

Die Umsetzung in Schritt (a) wird vorzugsweise bei erhöhter Temperatur durchgeführt, insbesondere bei Temperaturen im Bereich von 20 °C bis 180°C, bevorzugt im Bereich von 40 °C bis 100 °C, bevorzugter im Bereich von 55 °C bis 90 °C, noch bevorzugter im Bereich von 60 °C bis 90 °C, am bevorzugtesten bei 75 °C.

Die Umsetzung in Schritt (a) wird insbesondere entweder bei Eigendruck der Komponenten oder bei erhöhtem Druck durchgeführt. So wird die Umsetzung in Schritt (a) bevorzugt bei einem Druck im Bereich von 1 bis 16 bar, bevorzugter bei einem Druck im Bereich von 1 bis 15 bar, noch bevorzugter bei einem Druck im Bereich von 7 bis 14 bar, noch viel mehr bevorzugter bei 12 bar durchgeführt werden.

Ammoniak wird im Schritt (a) des erfindungsgemäßen Verfahrens bevorzugt als Reinstoff, d. h. als Gas zudosiert und liegt insbesondere während der Reaktion gelöst in Aceton bzw. gelöst im Reaktionsgemisch vor.

Aceton wird im Schritt (a) bevorzugt als Reinstoff zudosiert. Alternativ wird bevorzugt Aceton verwendet, was in Schritt (b) einer vorher durchlaufenen Runde des erfindungsgemäßen Verfahrens abgetrennt wurde und wie in Schritt (c) beschrieben rezykliert wird.

Daneben können im Acetonstrom **S_{Ac1}** in Schritt (a) neben Aceton und Ammoniak Kondensationsprodukte des Acetons mit sich selbst oder Ammoniak vorliegen können. Diese können aus vorhergehenden Umsetzungsschritten von Ammoniak und Aceton zu TAA stammen.

Der Schritt (a) des erfindungsgemäßen Verfahrens wird in Gegenwart eines Katalysators **K1** durchgeführt. Als Katalysator **K1** sind alle im Stand der Technik für diesen Reaktionstyp genannten Katalysatoren einsetzbar. Der Katalysator **K1** kann dabei homogen oder heterogen sein, ist bevorzugt jedoch heterogen.

Als homogener Katalysator **K1** sind alle im Stand der Technik für diesen Reaktionstyp beschriebenen homogenen Katalysatoren geeignet, z. B. Brönstedt-Säuren, Salze dieser Säuren oder Lewissäuren, wie sie in der EP 2 706 056 A1 beschrieben sind.

Der Begriff "Brönsted-Säuren" im Sinne der Erfindung umfasst insbesondere Salzsäure, Schwefelsäure, Salpetersäure, organische Säuren (RCOOH) oder Sulfonsäuren (RSO₃H), wobei R ausgewählt ist aus der Gruppe bestehend aus gesättigten, ungesättigten, verzweigten, unverzweigten, ringgeschlossenen, offenkettigen aliphatischen, aromatischen, substituierten, unsubstituierten Kohlenwasserstoffresten. Substituierte Kohlenwasserstoffreste im Sinne der Erfindung sind Kohlenwasserstoffreste, die mit Heteroatomen substituiert sind, insbesondere Kohlenwasserstoffreste, welche mit einem oder mehreren -OH, -NH, -CN, Alkoxy- und/oder Halogenresten substituiert sind, bevorzugt mit einem oder mehreren Halogenresten substituiert sind, besonders bevorzugt mit einem oder mehreren Resten ausgewählt aus F, Cl, Br und I substituiert sind, ganz besonders bevorzugt mit einem oder mehreren Resten ausgewählt aus F und Cl substituiert sind.

"Salze einer Brönstedtsäure" im Sinne der Erfindung sind insbesondere Ammoniumsalze (d. h. Salze mit Ammoniak, Aminen, Hydrazinen, Hydroxylaminen) oder Phosphoniumsalze (d. h. Salze mit Phosphanen). Lewis-Säuren im Sinne der Erfindung sind insbesondere Verbindungen der 4. bzw. der 13. Gruppe des Periodensystems, bevorzugt Halogenide (AlCl₃, BF₃, TiCl₄), Alkoxide [Al(OR*)₃, B(OR*)₃, Ti(OR*)₄] oder Alkylverbindungen (z.B. AlR*₃), wobei R* ausgewählt ist aus der Gruppe bestehend aus gesättigten, ungesättigten, verzweigten, unverzweigten, ringgeschlossenen, offenkettigen aliphatischen, aromatischen, substituierten, unsubstituierten Kohlenwasserstoffresten.

Lewis-Säuren im Sinne der Erfindung sind auch Salze Lewis-saurer Alkali- bzw. Erdalkalimetalle (z.B. CaCl₂, MgCl₂, LiCI).

Vorzugsweise ist in den Fällen, in denen **K1** ein homogene Katalysator ist, dieser ausgewählt aus der Gruppe der Ammoniumsalze, insbesondere aus der Gruppe umfassend Salze aus Ammoniak und starken Brönsted-Säuren [z. B. Salzsäure, Schwefelsäure, Salpetersäure, organische Säuren (R**COOH) oder Sulfonsäuren (R**SO₃H), wobei R** ausgewählt ist aus der Gruppe bestehend aus gesättigten, ungesättigten, verzweigten, unverzweigten, ringgeschlossenen, offenkettigen aliphatischen, aromatischen, substituierten, unsubstituierten Kohlenwasserstoffresten].

Bevorzugt ist in den Fällen, in denen **K1** ein homogener Katalysator ist, dieser dann Ammoniumnitrat. Ammoniumnitrat hat den Vorteil, dass es günstig, nicht toxisch, halogenidfrei und damit weniger korrosiv ist.

Bevorzugt wird als Katalysator **K1** jedoch ein heterogener Katalysator, insbesondere ein fester, saurer Katalysator eingesetzt, wie er beispielsweise in der DE 28 07 172 A1, der CN 103224465 A oder der DE 10 2010 062 804 A1 beschrieben ist. Dies sind solche Katalysatoren, die in dem Reaktionsmedium praktisch unlöslich sind. Bevorzugt wird dazu ein Katalysator genutzt, der anorganisch oder organisch ist und aktive saure Gruppen, bevorzugt Sulfonsäureestergruppen oder Phosphorsäureestergruppen, aufweist. Der Begriff "Sulfonsäure**ester**gruppen" bzw. "Phosphorsäure**ester**gruppen" bezieht sich auf die Bindung des Schwefel- bzw. Phosphoratoms über ein Sauerstoffatom an den jeweiligen Träger. Es versteht sich von selbst, dass im Sinne der Erfindung eine Sulfonsäureestergruppe eine saure "-OS(O)OH"-Gruppe aufweist und eine Phosphorsäureestergruppe eine saure "-OP(O)(OH)₂-Gruppe" aufweist, die zur "-OS(O)O⁻"-Gruppe bzw. zur "-OP(O)(O⁻)₂-Gruppe" deprotonieren kann.

**K1** ist demnach insbesondere ausgewählt aus der Gruppe bestehend aus Aluminiumhydrosilikaten vom Betonit- und/oder Montmorillonit-Typ, anorganische Ionenaustauscher auf Aluminiumsilikat-Basis vom Zeolithtyp, Mordenittyp, Erionittyp oder auch mit Phosphorsäure bei 700 bis 1100 °C, wie in CA 772 201 beschrieben, behandelte Diatomerde.

Besonders für **K1** bevorzugte heterogene Katalysatoren sind lonenaustauscherharze, insbesondere Kationenaustauscherharze. Diese sind bevorzugt sauer.

Als lonenaustauscherharze für **K1** kommen insbesondere solche auf anorganischer (beispielsweise Siliciumdioxid) oder organischer (beispielsweise Polystyrol oder Polystyrolcopolymere, wie Styrol-Divinylbenzol-Copolymere), bevorzugt organischer, Basis, die protische Säuregruppen, insbesondere Alkylsulfonsäureestergruppen, Sulfonsäureestergruppen (-SO₃⁻), Phosphorsäureestergruppen, insbesondere Sulfonsäureestergruppen aufweisen, in Frage.

Ein besonders bevorzugter heterogener Katalysator für **K1** ist ausgewählt aus der folgenden Gruppe:
- Ein Katalysator, der ein Polymerharz, insbesondere Polystyrol oder Styrol-Divinylbenzol-Copolymer, bevorzugt Polystyrol, und protische Säuren, insbesondere -SO₃⁻-Gruppen, als funktionelle Gruppen aufweist (käuflich erwerblich als "Amberlyst^{®} 15", "Amberlite^{®} 200", "Lewatit^{®} SP 120" bzw. "Lewatit^{®} K2621"); daneben ist auch Polystyrolsulfonat mit der CAS-Nummer: 28210-41-5 einsetzbar;
- Ein Katalysator, der protische Säuren, insbesondere Sulfonsäure in polymerer Form aufweist und perfluoriert sein kann (beschrieben in DE 10 2010 062 804 A1, US 4,831,146). Dieser kann zum Beispiel ein sulfoniertes Tetrafluorethylen (CAS-Nummer: 31175-20-9) oder eine feste geträgerte perfluorierte Sulfonsäure in polymerer Form mit Siliciumdioxid als Trägermaterial sein. Derartige Katalysatoren sind unter anderem unter den Handelsnamen Nafion^{®}, Aciplex^{®} F, Femion^{®}, Neosepta^{®}, Fumion^{®} F erhältlich. Ein bevorzugter Katalysator ist Nafion^{®} SAC-13. Bei Nafion^{®} SAC-13 handelt es sich um poröse Siliciumdioxidpartikel, auf dem Nafion^{®} in einer Beladung von etwa 13 Gew.-% adsorbiert worden ist;
- Poly(2-acrylamido-2-methyl-1-propanesulfonsäure), vertrieben als PolyAMPS^{®} von Lubrizol.

Am bevorzugtesten wird als **K1** ein heterogener Katalysator eingesetzt, der ein Polymerharz, insbesondere Polystyrol oder Styrol-Divinylbenzol-Copolymer, bevorzugt Polystyrol, und protische Säuren, insbesondere -SO₃⁻-Gruppen, als funktionelle Gruppen aufweist (käuflich erwerblich als "Amberlyst^{®} 15", "Amberlite^{®} 200", "Lewatit^{®} SP 120" bzw. "Lewatit^{®} K2621").

Die Einsatzverhältnisse der Edukte im Schritt (a) des erfindungsgemäßen Verfahrens können in weiten Bereichen gewählt werden, insbesondere wird Aceton im Überschuss zu Ammoniak eingesetzt. Bevorzugt beträgt das Molverhältnis von in Schritt (a) eingesetztem Aceton zu in Schritt (a) eingesetztem Ammoniak 3 : 1 bis 20 : 1, wobei ein Verhältnis von 6 : 1 bis 10 : 1 bevorzugt ist und ein Verhältnis von 7 : 1 besonders bevorzugt ist.

Die eingesetzte Menge an Katalysator **K1** ist nicht besonders beschränkt und kann vom Fachmann bestimmt werden. Typischerweise kann, wenn der Katalysator ein homogener aus der Gruppe der Brönstedt-Säuren, Salze dieser Säuren oder Lewissäuren, bevorzugt ein Ammoniumsalz, noch bevorzugter Ammoniumnitrat ist, dieser im Molverhältnis von Ammoniak zu Katalysator, vorzugsweise Ammoniumnitrat, im Bereich von 1 : 0.8 bis 1 : 0.02 eingesetzt werden. Ganz besonders bevorzugt liegt das Molverhältnis von Aceton : Ammoniak : Ammoniumnitrat im Bereich von 7 bis 8 : 0.9 bis 1.1 : 0.085 bis 0.098.

In der bevorzugten Ausführungsform, in welcher ein fester, saurer Ionenaustauscher für **K1** eingesetzt wird, kann dieser als Festbett eingesetzt werden, beispielsweise in einer Menge von 10 bis 20 Vol.-% bezogen auf die Gesamtmenge des in Schritt (a) eingesetzten Acetons und - falls ein solches eingesetzt wurde - des Mesityloxids.

Im Anschluss an Schritt (a) des erfindungsgemäßen Verfahrens wird dann ein Rohproduktstrom **S_{RP1}** erhalten, welches neben dem gewünschten Produkt Triacetonamin auch das ursprünglich eingesetzte Edukt Aceton, sowie gegebenenfalls Ammoniak und gegebenenfalls als Nebenprodukt Mesityloxid und gegebenenfalls mindestens ein Nebenprodukt ausgewählt aus der Gruppe bestehend aus Diacetonalkohol, Diacetonamin, Acetonin, Phoron, Isophoron, bevorzugt ausgewählt aus der Gruppe bestehend aus Diacetonalkohol, Diacetonamin, Acetonin, Phoron, Isophoron, umfasst.

Der Anteil des TAA, Aceton, Mesityloxid, Wasser und der genannten Nebenprodukte in **RP1** ist nicht weiter beschränkt und ergibt sich aus der Stöchiometrie und den speziellen Reaktionsbedingungen. Der Anteil der jeweiligen Verbindung kann per GC bestimmt werden. Beispielsweise liegt nach der Reaktion ein Gemisch vor, in dem der Gehalt an Aceton bei 55 bis 60 Gew.-%, an Mesityloxid bei 10 Gew.-%, an TMDH-Pyridin bei 5 bis 6 Gew.-%, an der Summe aus Diacetonamin, Diacetonalkohol und Phoron bei 4 bis 6 Gew.-%, an TAA bei 14 bis 16 Gew.-%, und an höher als TAA siedenden Komponenten (zum Beispiel Iso-Phoron) bei 3 bis 4 Gew.-% liegt, und der Anteil von Wasser 7 Gew.-% ist.

### 2. Schritt (b)

Im Schritt (b) des erfindungsgemäßen Verfahrens wird mindestens teilweise Aceton aus **S_{RP1}** abgetrennt. Dies erfolgt destillativ, bevorzugt in einer Destillationskolonne.

Dadurch wird ein gasförmiger Strom **S_{Ac2}** umfassend Aceton erhalten. **S_{Ac2}** umfasst gegebenenfalls auch Ammoniak und/oder Mesityloxid. **S_{Ac2}** umfasst, falls der Rohproduktstrom **S_{RP1}** Ammoniak umfasst, bevorzugt auch Ammoniak.

Gegebenenfalls findet während oder vor, bevorzugt vor, dem Schritt (b) auch eine Abtrennung des Katalysators **K1** statt. Dies kann durch Zugabe einer Base geschehen. Zum Beispiel wird NaOH zugegeben, wenn **K1** ein Ammoniumsalz ist, und das dann ausgefallene Natriumnitrat wird anschließend abgetrennt.

Bei Verwendung eines heterogenen Katalysators erübrigt sich ein separater Reinigungsschritt oder ist zumindest deutlich einfacher, da beispielsweise bei Verwendung eines Festbettkatalysators dieser Katalysator im Reaktor verbleibt oder in anderen Fällen im Reaktionskessel verbleiben kann und/ oder durch Filtration abgetrennt werden kann. Auch deshalb ist die Verwendung eines heterogenen Katalysators für **K1** vorzuziehen.

Schritt (b) wird insbesondere bei einem Druck von 0.5 bis 2 bar, bevorzugt bei Normaldruck durchgeführt.

Bevorzugt wird Schritt (b) bei einer Temperatur durchgeführt, die oberhalb der Siedetemperatur von Aceton und Mesityloxid liegt, aber unterhalb der Siedetemperatur der typischen Nebenprodukte, was der Fall ist, wenn sie unterhalb der Siedetemperatur von Diacetonalkohols liegt, was das niedrigstsiedende der üblichen Nebenprodukte ist.

Noch bevorzugter wird Schritt (b) bei einer Temperatur durchgeführt, die oberhalb der Siedetemperatur von Aceton liegt, aber unterhalb der Siedetemperatur von Mesityloxid (und damit auch unterhalb der Siedetemperatur von Diacetonalkohol, Diacetonamin, TMDH-pyridin, Acetonin, Phoron, Triacetonamin, Isophoron) liegt.

Die Siedepunkte der typischen Nebenprodukte Diacetonalkohol (CAS-Nummer 123-42-2; Siedepunkt bei Normaldruck: 166 °C), Acetonin (CAS-Nummer 556-72-9; Siedepunkt ~ 170 °C), Diacetonamin (CAS-Nummer 625-04-7, Siedepunkt ~ 180 °C), Phoron (CAS-Nummer 504-20-1; Siedepunkt bei Normaldruck: 197 °C) liegen zwischen den Siedepunkten des Acetons (CAS-Nummer 67-64-1; Siedepunkt bei Normaldruck: 56 °C) bzw. Mesityloxids (CAS-Nummer 141-79-7; Siedepunkt bei Normaldruck: 130 °C) und des TAAs (Siedepunkt bei Normaldruck: 205 °C), und im Falle des Isophorons (CAS-Nummer 78-59-1; Siedepunkt bei Normaldruck: 215 °C) auch darüber.

Da der Siedepunkt von Ammoniak unterhalb des Siedepunktes von Aceton liegt, ist es möglich, dass in Schritt (b) zusammen mit Aceton auch gegebenenfalls im Rohproduktstrom **S_{RP1}** enthaltener Ammoniak mit abgetrennt wird. In dieser Ausführungsform umfasst der erhaltene gasförmige Strom **S_{Ac2}** neben Aceton auch Ammoniak. Dies ist jedoch vorteilhaft, da im Folgeschritt (c) dann auch Ammoniak mit in den flüssigen Acetonstrom **S_{Ac3}** absorbiert wird, und so weniger frischer Ammoniak mit in das System eigespeist werden muss.

In einer bevorzugten Ausführungsform der vorliegenden Erfindung wird vor und/oder während der destillativen Abtrennung des Acetons aus Rohproduktstrom **S_{RP1}** gemäß Schritt (b) Wasser zu Rohproduktstrom **S_{RP1}** zugegeben wird und mindestens eines der Nebenprodukte in Rohproduktstrom **S_{RP1}** ausgewählt aus der Gruppe bestehend aus Phoron, Diacetonalkohol, Diacetonamin, Acetonin, Isophoron, bevorzugt ausgewählt aus der Gruppe bestehend aus Diacetonalkohol, Diacetonamin, Acetonin, Isophoron, wird mindestens teilweise mit Wasser umgesetzt, so dass mindestens eines der Nebenprodukte in Rohproduktstrom **S_{RP1}** mindestens teilweise in Aceton gespalten wird. Dies erhöht noch weiter die Effizienz des erfindungsgemäßen Verfahrens, da somit mehr und leichter abzutrennendes Aceton erhalten wird.

Die Zugabe von Wasser zu **S_{RP1}** erfolgt in der bevorzugten Ausführungsform vor und/oder während der in Schritt (b) stattfindenden destillativen Abtrennung von Aceton aus **S_{RP1}**. Es versteht sich von selbst, dass die Zugabe von Wasser "vor der Abtrennung von Aceton aus **S_{RP1}**" bedeutet, dass dieser Zeitpunkt zwischen den Schritten (a) und (b) liegt, da erst nach Schritt (a) das Rohprodukt **S_{RP1}** erhalten wird.

Durch die Zugabe von Wasser wird das Gleichgewicht von der Seite der Nebenprodukte auf die der Spaltprodukte derselben, also neben Aceton vor allem Mesityloxid, verschoben wird. Um diese Verschiebung des Gleichgewichts ausreichend zu gewährleisten, genügt nicht das Wasser, welches aus der Umsetzung in Schritt (a) resultiert und noch mindestens teilweise in **S_{RP1}** vorliegen kann. Es ist vielmehr nötig, zumindest eine bestimmte Menge zusätzlichen Wassers zu **S_{RP1}** zu geben, um das Gleichgewicht in der folgenden Umsetzung auf die Seite des gewünschten Produkts Aceton zu schieben.

Insbesondere wird in dieser bevorzugten Ausführungsform des erfindungsgemäßen Verfahrens Wasser in einer Menge von ≥ 0.1 Gewichts.-%, bevorzugt ≥ 0.5 Gew.-%, bevorzugter ≥ 1 Gew.-%, noch bevorzugter ≥ 5 Gew.-%, noch mehr bevorzugter im Bereich von 5 bis 40 Gew.-%, noch viel mehr bevorzugter im Bereich von 10 bis 20 Gew.-% zugegeben, jeweils bezogen auf die Summe der Gewichte des von **S_{RP1}** umfassten Phorons, Diacetonalkohols, Diacetonamins, Acetonins und Isophorons. Dieser Anteil kann vom Fachmann mit Hilfe von Gaschromatographie ermittelt werden. Das Gesamtgewicht von **S_{RP1}** kann bei kontinuierlichen Verfahren durch Ermittlung des Durchflusses (Massedurchflußmesser) ermittelt werden.

Alternativ kann in dieser bevorzugten Ausführungsform des erfindungsgemäßen Verfahrens Wasser in einer Menge von ≥ 1 Gewichts.-%, bevorzugt ≥ 3 Gew.-%, bevorzugter ≥ 4 Gew.-%, noch bevorzugter ≥ 5 Gew.-%, noch mehr bevorzugter im Bereich von 5 bis 40 Gew.-%, noch viel mehr bevorzugter im Bereich von 10 bis 20 Gew.-%, jeweils bezogen auf das Gesamtgewicht der in Schritt (a) des erfindungsgemäßen Verfahrens eingesetzten Menge an Aceton, zugegeben werden.

Die Umsetzung des zugegebenen Wassers in der bevorzugten Ausführungsform mit mindestens einem der Nebenprodukte in **S_{RP1}** ausgewählt aus der Gruppe bestehend aus Phoron, Diacetonalkohol, Diacetonamin, Acetonin, Isophoron, bevorzugt ausgewählt aus der Gruppe bestehend aus Diacetonalkohol, Diacetonamin, Acetonin, Isophoron, zu Aceton kann dann unter den dem Fachmann geläufigen Bedingungen durchgeführt werden. Es handelt sich dabei um eine Hydrolyse der Nebenprodukte zu Aceton. Der dazu eingesetzte Temperaturbereich ist insbesondere < 205 °C, bevorzugt < 204 °C, liegt bevorzugter im Bereich von 30 °C bis 200 °C, liegt noch bevorzugter im Bereich von 70 °C bis 185 °C.

Bevorzugt wird gemäß der bevorzugten Ausführungsform des erfindungsgemäßen Verfahrens das Wasser zu **S_{RP1}** zugegeben, während in Schritt (b) Aceton aus **S_{RP1}** mindestens teilweise destillativ abgetrennt werden.

Noch bevorzugter ist diese Ausführungsform dann, wenn die mindestens teilweise destillative Abtrennung von Aceton aus **S_{RP1}** in Schritt (b) in einer Destillationskolonne erfolgt.

Noch viel mehr bevorzugter erfolgt die Umsetzung des zugegebenen Wassers in dieser bevorzugten Ausführungsform mit mindestens einem der Nebenprodukte in **S_{RP1}** ausgewählt aus der Gruppe bestehend aus Phoron, Diacetonalkohol, Diacetonamin, Acetonin, Isophoron, bevorzugt ausgewählt aus der Gruppe bestehend aus Diacetonalkohol, Diacetonamin, Acetonin, Isophoron, zu Aceton dann in der Gasphase.

In diesem Fall wird insbesondere das Wasser in die Destillationskolonne während der destillativen Abtrennung, etwa über einen Zulauf von Wasser in die Destillationskolonne oder durch Zugabe von Wasserdampf zur Destillationskolonne, zugeführt. Die Umsetzung mindestens eines der Nebenprodukte Diacetonalkohol, Diacetonamin, Acetonin, Phoron, Isophoron, bevorzugt der Nebenprodukte ausgewählt aus der Gruppe bestehend aus Diacetonalkohol, Diacetonamin, Acetonin, Isophoron, mit Wasser zu Aceton findet dann in der Destillationskolonne statt.

Diese Ausführungsform hat sich als besonders vorteilhaft erwiesen, da diese Ausführungsform besonders gut gewährleistet, dass gezielt die Nebenkomponenten zersetzt werden und die unerwünschte Rückreaktion des TAA unterdrückt wird. Dies wird besonders gut dann gewährleistet und es ist deshalb bevorzugt, wenn der bei der Destillation in Schritt (b) eingesetzte Temperaturbereich unterhalb der Siedetemperatur von TAA, wobei der Druck insbesondere Normaldruck ist, liegt, bevorzugt < 205 °C bei Normaldruck beträgt, bevorzugter < 204 °C bei Normaldruck beträgt, noch bevorzugter im Bereich von 30 °C bis 200 °C bei Normaldruck liegt, noch mehr bevorzugter im Bereich von 70 °C bis 185 °C bei Normaldruck liegt.

Die Abtrennung von TAA aus dem Destillationsrückstand des Schrittes (b) des erfindungsgemäßen Verfahrens kann im Übrigen durch Kristallisation, Destillation, bevorzugt mit Destillation, welche noch bevorzugter in einer nachfolgenden Destillationskolonne stattfindet, erfolgen.

### 3. Schritt (c)

Die Erfindung beruht nun drauf, dass der in Schritt (b) erhaltene gasförmiger Strom **S_{Ac2}** mindestens teilweise im Gegenstrom in einen flüssigen Acetonstrom **S_{Ac3}** absorbiert wird.

"Mindestens teilweise" bedeutet im Sinne der Erfindung, dass im Schritt (c) entweder das gesamte oder nur ein Teil des Acetons im in Schritt (b) erhaltenen gasförmigen Strom **S_{Ac2}** im Gegenstrom in einen flüssigen Acetonstrom **S_{Ac3}** absorbiert wird. Bevorzugt wird im Schritt (c) nur ein Teil des Acetons im in Schritt (b) erhaltenen gasförmigen Strom **S_{Ac2}** in einen flüssigen Acetonstrom **S_{Ac3}** absorbiert und der übrige Teil kondensiert. In dieser bevorzugten Ausführungsform werden im Schritt (c) noch bevorzugter 1 bis 99 Gew.-%, noch bevorzugter 20 bis 90 Gew.-%, noch mehr bevorzugter 30 bis 80 Gew.-%, noch viel mehr bevorzugter 50 bis 70 Gew.-% des Acetons im in Schritt (b) erhaltenen gasförmigen Strom **S_{Ac2}** kondensiert und der Rest des Acetons im in Schritt (b) erhaltenen gasförmigen Strom **S_{Ac2}** im Gegenstrom in den flüssigen Acetonstrom **S_{Ac3}** absorbiert.

Beim Acetonstrom **S_{Ac3}** handelt es sich üblicherweise um Aceton, was aufgrund des Verbrauchs in der Reaktion zum TAA dem System zugefügt werden muss.

Im Gegensatz zu herkömmlichen Verfahren, in welchem aus dem Rohprodukt destilliertes Aceton kondensiert und dann gegebenenfalls dem Prozess wieder zugeführt wird, hat dies den Vorteil, dass die Wiedergewinnung des Acetons einfach möglich ist und die Energie für die Kondensation und Rückführung des aus dem Rohproduktstrom **S_{RP1}** isolierten Acetons nicht aufgebracht werden muss.

Schritt (c) wird bevorzugt in einem dem Fachmann bekannten Gaswäscher (Gasstripper) durchgeführt.

In Schritt (c) wird insbesondere ein flüssiger Acetonstrom **S_{Ac3}** typischerweise von oben nach unten geführt, während der gasförmiger Strom **S_{Ac2}** mindestens teilweise in die Gegenrichtung geführt wird und den flüssiger Acetonstrom **S_{Ac3}** kontaktiert, so dass der flüssige Acetonstrom **S_{Ac3}** den gasförmigen Stroms **S_{Ac2}** mindestens teilweise absorbiert.

In Schritt (c) weist der gasförmiger Strom **S_{Ac2}** bevorzugt eine Temperatur auf, die mindestens 1 °C über der Siedetemperatur des Acetons beim jeweiligen Druck liegt, bei Normaldruck demnach insbesondere im Bereich von 57 °C bis 100 °C, bevorzugt im Bereich von 58 °C bis 80 °C, bevorzugter im Bereich von 59 °C bis 70 °C, noch bevorzugter im Bereich von 60 °C bis 65 °C liegt.

In Schritt (c) weist der flüssige Acetonstrom **S_{Ac3}** bevorzugt eine Temperatur auf, die mindestens 10 °C unterhalb der Siedetemperatur des Acetons beim jeweiligen Druck liegt, bei Normaldruck demnach insbesondere im Bereich von -10 °C bis 40 °C, bevorzugter im Bereich von 0 °C bis 30 °C, bevorzugter im Bereich von 5 °C bis 25 °C, noch bevorzugter im Bereich von 8 °C bis 20 °C, noch bevorzugter im Bereich von 10 °C bis 15 °C, am bevorzugtesten bei 12 °C liegt.

Dies garantiert am besten, dass der nach Schritt (c) erhaltene Acetonstrom **S_{Ac4}** flüssig vorliegt und andererseits, dass der gasförmige Strom **S_{Ac2}** nach der Destillation nicht zu sehr gekühlt werden muss, was einen höheren Energieaufwand nötig machen würde.

Im Anschluss an Schritt (c) des erfindungsgemäßen Verfahrens wird dann ein flüssiger Acetonstrom **S_{Ac4}** erhalten, welches aus dem flüssiger Acetonstrom **S_{Ac3}** und dem aus de, gasförmige Strom **S_{Ac2}** absorbierten Aceton zusammensetzt.

### 4. Schritt (d)

In Schritt (d) des erfindungsgemäßen Verfahrens wird der flüssige Acetonstrom **S_{Ac4}** dann wieder in einer weiteren Umsetzung mit einem Ammoniakstroms **S_{Am1}** wie in Schritt (a) beschrieben umgesetzt.

Die nachfolgenden Beispiele sollen die Erfindung illustrieren, ohne diese zu beschränken.

### Beispiel 1 (erfindungsgemäß)

Zwei in Reihe geschaltete zylindrische Reaktoren wurden mit Lewatit K2621 (Polystyrol-Katalysator mit -SO₃⁻ als funktionelle Gruppe; von Lanxess) so gefüllt, dass dieses Katalysatormaterial durch Siebe oben und unten begrenzt, angeordnet war. Das Schüttvolumen des Katalysators betrug je 600 mL im wasserfeuchten Zustand. Der Reaktor wurde kontinuierlich mit 630 g/h Aceton und 25 g/h Ammoniak beschickt. Dabei wurde eine Temperatur von 75 °C und ein Druck von 14 bar eingestellt. Der Zulauf betrug insgesamt 961.83 g (16.58 mol) Aceton. Die LHSV (liquid hourly space velocity, also das Volumen an Reaktanden die pro Stunde bezogen auf das Reaktorvolumen zugegeben wurden), betrug im Falle des Ammoniaks 0.03 bis 0.06 h⁻¹, und im Falle des Acetons 0.66 bis 1.33 h⁻¹.

1 kg des Reaktoraustrages wurde mit 3 bis 5 Gew.-% Wasser, bezogen auf die eingesetzte Menge an Aceton, versetzt und anschließend destillativ bei Normaldruck an einer Rektifikationskolonne aufgearbeitet.

Dabei wurde so lange destilliert, dass eine Abtrennung sämtlicher Leichtsieder (Aceton, Mesityloxid, Diacetonalkohol, Diacetonamin) gelang, TMDHPyridin und TAA und weitere entstandene Hochsieder jedoch als Abfall- bzw. Wertprodukt über den Sumpf abgetrennt wurden. Zwischensieder, wie Acetonin und Phoron, wurden zu einem möglichst hohen Anteil in leichtsiedende Komponenten hydrolisiert und waren dadurch abtrennbar.

Das in der Destillationskolonne abgetrennte Aceton wurde gasförmig im Gegenstrom in einem Gasstripper mit einem flüssigen Strom von frischem Aceton, welches eine Temperatur von 12 °C aufwies, geleitet. Dabei wurde das gasförmige Aceton in das flüssige Aceton absorbiert und der danach erhaltene flüssige Mischstrom zur weiteren Umsetzung zu TAA in die Reaktoren eingeleitet.

Diese Vorgehensweise erbrachte eine deutliche Verringerung des Energieaufwandes gegenüber der separaten Kondensation des abgetrennten Acetons und Vermischung mit zusätzlichem Frischaceton.

Durch diese Energieersparnis war eine deutliche Effizienzsteigerung des Verfahrens möglich.

### Vergleichsbeispiel (nicht erfindungsgemäß, entspricht Beispiel 5/5 a aus der Offenlegungsschrift DE 28 07 172 A1)

Ein zylindrischer Reaktor wurde mit Lewatit ^{®} SP 120 so gefüllt, dass das Katalysatormaterial durch Siebe abgetrennt zwischen zwei Lagen von Tonraschigringen angeordnet war. Das Schüttvolumen des Katalysators betrug 650 ml im feuchten Zustand, schrumpfte aber nach Behandlung mit Aceton auf 500 ml. Der Reaktor wurde mittels elektrischer Beheizung auf 100 °C Reaktionstemperatur gebracht und dann kontinuierlich mit 1000 ml/h Aceton und 50 ml/h Ammoniak beschickt, indem die beiden Reaktanten aus Vorlagen flüssig in den unteren Reaktorteil gepumpt wurden. Das Reaktionsprodukt gelangte vom oberen Reaktorteil flüssig in einen Abscheider und wurde auf diesem kontinuierlich abgezogen; das System wurde mit Inertgas (Hp) unter einem Druck von 70 bar gehalten.

Die destillative Aufarbeitung des Rohprodukts erfolgte wie auf Seite 12 der DE 28 07 172 A1 beschrieben, durch destillative Auftrennung des Rohproduktes. Die ersten drei Fraktionen umfassend Aceton, Mesityloxid, Ammoniak (als Zersetzungsprodukt des Diacetonamins) wurden zusammen mit Frischaceton als Edukt eingesetzt.

Die sich bei kontinuierlichem Betrieb einstellende Zusammensetzung des erhaltenen Rohprodukts war wie in DE 28 07 172 A1 beschrieben: 64.5 Gew.-% Aceton, 4.6 Gew.-% Mesityloxid, 4.0 Gew.-% Diacetonamin, 7.6 Gew.-% stickstoffhaltige Zwischenfraktionen, 18.8 Gew.-% Triacetonamin und 0.5 Gew.-% Höherssieder.

Bei der kontinuierlichen Durchführung konnte durch diese Ausführung eine Ausbeute an TAA von 69.9 % erhalten werden (bezogen auf die Menge des insgesamt eingesetzten Acetons und Umsatz).

### Beispiel 2 (erfindungsgemäß)

Das in Vergleichsbeispiel beschriebene Vorgehen wurde mit folgendem Unterschied wiederholt:
Die den ersten drei Fraktionen entsprechenden Destillatströme wurden im Gegenstrom mit frischem gekühltem Aceton vermengt und dann als Edukt wiedereingesetzt. Bei kontinuierlicher Durchführung dieses Verfahrens stellte sich eine Zusammensetzung des Rohprodukts von 63.5 Gew.-% Aceton, 10.4 Gew.-% Mesityloxid, 6.3 Gew.-% Diacetonamin, 4.9 Gew.-% stickstoffhaltige Zwischenfraktionen, 13.7 Gew.-% Triacetonamin und 1.2 % Höherssieder ein.

Es stellte sich somit überraschend heraus, dass die TAA Konzentration im Rohprodukt zwar niedriger als jene im Rohprodukt nach dem Vergleichsbeispiel ist, dass aber die Gesamtausbeute (bezogen auf die Menge des insgesamt eingesetzten Acetons und Umsatz) an TAA auf 73.6 % erhöht werden konnte.

Es wird vermutet, dass der Grund dafür darin liegt, dass das Rohprodukt gemäß Beispiel 2 gegenüber jenem des Vergleichsbeispiels einen höheren Anteil an Produkten wie Mesityloxid, Aceton, Diacetonamin aufweist, welche wieder als Edukte eingesetzt werden können. Außerdem ist der Anteil der stickstoffhaltigen Zwischenfraktionen im Rohprodukt nach Beispiel 2 geringer als jener des im Vergleichsbeispiel erhaltenen Rohprodukts. Diese stickstoffhaltigen Zwischenprodukte (das heißt höhermolekulare Produkte wie TMDH-Pyridin) können nicht erneut als Edukt eingesetzt werden, und sind demnach für weitere Umsetzungen verloren.

Im erfindungsgemäßen Verfahren wird demnach im Vergleich zu den Verfahren des Standes der Technik, insbesondere jenem der DE 28 07 172 A1, ein vorteilhaftes Nebenproduktspektrum im Rohprodukt erhalten. Gleichzeitig kann dadurch ein höherer Anteil an wiederverwertbaren Nebenprodukten auf einfache, energieeffiziente Weise der Reaktion wieder zugeführt werden. Dies führt, bezogen auf die Menge des insgesamt eingesetzten Acetons, zu einer höheren Ausbeute an TAA.

## Patentansprüche

1. Verfahren zur Herstellung von Triacetonamin, umfassend die folgenden Schritte:
(a) Umsetzung eines Acetonstroms **S_{Ac1}** und eines Ammoniakstroms **S_{Am1}** in Gegenwart eines Katalysators **K1** zu einem Rohproduktstrom **S_{RP1}** umfassend Triacetonamin, Aceton,
(b) mindestens teilweise destillative Abtrennung von Aceton aus **S_{RP1}**, wodurch ein gasförmiger Strom **S_{Ac2}** umfassend Aceton erhalten wird,
(c) mindestens teilweise Absorption des gasförmigen Stroms **S_{Ac2}** im Gegenstrom in einen flüssigen Acetonstrom **S_{Ac3}**, wodurch ein flüssiger Acetonstrom **S_{Ac4}** erhalten wird,
(d) Wiederholung des Schrittes (a), wobei der Acetonstrom **S_{Ac4}** als Acetonstrom **S_{Ac1}** eingesetzt wird.

2. Verfahren nach Anspruch 1, wobei die in Schritt (b) stattfindende mindestens teilweise destillative Abtrennung von Aceton aus **S_{RP1}** in einer Destillationskolonne erfolgt.

3. Verfahren nach Anspruch 1 oder 2, wobei der bei der Destillation in Schritt (b) eingesetzte Temperaturbereich unterhalb der Siedetemperatur von Triacetonamin liegt.

4. Verfahren nach einem der Ansprüche 1 bis 3, wobei vor und/oder während der destillativen Abtrennung des Acetons aus Rohproduktstrom **S_{RP1}** gemäß Schritt (b) Wasser zu Rohproduktstrom **S_{RP1}** zugegeben wird und mindestens eines der Nebenprodukte in Rohproduktstrom **S_{RP1}** ausgewählt aus der Gruppe bestehend aus Phoron, Diacetonalkohol, Diacetonamin, Acetonin, Isophoron, mindestens teilweise mit Wasser umgesetzt wird, so dass mindestens eines der Nebenprodukte in Rohproduktstrom **S_{RP1}** mindestens teilweise in Aceton gespalten wird.

5. Verfahren nach einem der Ansprüche 1 bis 4, wobei der Schritt (a) bei einer Temperatur von 20 °C bis 180 °C durchgeführt wird.

6. Verfahren nach einem der Ansprüche 1 bis 5, wobei das Molverhältnis von in Schritt (a) eingesetztem Aceton zu in Schritt (a) eingesetztem Ammoniak 3 : 1 bis 20 : 1 beträgt.

7. Verfahren nach einem der Ansprüche 1 bis 6, wobei **K1** ein heterogener Katalysator ist.

8. Verfahren nach einem der Ansprüche 1 bis 7, wobei in Schritt (c) ein Teil des Acetons im in Schritt (b) erhaltenen gasförmigen Strom **S_{Ac2}** in den flüssigen Acetonstrom **S_{Ac3}** absorbiert und der übrige Teil kondensiert wird.

9. Verfahren nach einem der Ansprüche 1 bis 8, welches kontinuierlich durchgeführt wird.

## Claims

1. Process for preparing triacetonamine, comprising the following steps:
(a) reaction of an acetone stream **S_{Ac1}** and an ammonia stream **S_{Am1}** in the presence of a catalyst **K1** to give a crude product stream **S_{RP1}** comprising triacetonamine, acetone,
(b) at least partial distillative removal of acetone from **S_{RP1}** to obtain a gaseous stream **S_{Ac2}** comprising acetone,
(c) at least partial absorption of the gaseous stream **S_{Ac2}** in countercurrent into a liquid acetone stream **S_{Ac3}** to obtain a liquid acetone stream **S_{Ac4},**
(d) repetition of step (a), with the acetone stream **S_{Ac4}** being used as the acetone stream **S_{Ac1}.**

2. Process according to Claim 1, wherein the at least partial distillative removal of acetone from **S_{RP1}** which takes place in step (b) is performed in a distillation column.

3. Process according to Claim 1 or 2, wherein the temperature range used in the distillation in step (b) is below the boiling point of triacetonamine.

4. Process according to any of Claims 1 to 3, wherein, prior to and/or during the distillative removal of the acetone from crude product stream **S_{RP1}** as per step (b), water is added to crude product stream **S_{RP1}** and at least one of the by-products in crude product stream **S_{RP1}**, selected from the group consisting of phorone, diacetone alcohol, diacetonamine, acetonin, and isophorone, is at least partially reacted with water so that at least one of the by-products in crude product stream **S_{RP1}** is at least partially cleaved into acetone.

5. Process according to any of Claims 1 to 4, wherein step (a) is carried out at a temperature of 20°C to 180°C.

6. Process according to any of Claims 1 to 5, wherein the molar ratio of acetone used in step (a) to ammonia used in step (a) is 3:1 to 20:1.

7. Process according to any of Claims 1 to 6, wherein **K1** is a heterogeneous catalyst.

8. Process according to any of Claims 1 to 7, wherein in step (c) a portion of the acetone in the gaseous stream **S_{Ac2}** obtained in step (b) is absorbed into the liquid acetone stream **S_{Ac3}** and the remaining portion is condensed.

9. Process according to any of Claims 1 to 8, wherein said process is carried out continuously.

## Revendications

1. Procédé de préparation de triacétonamine, comprenant les étapes suivantes :
(a) réaction d'un courant d'acétone **S_{Ac1}** et d'un courant d'ammoniac **S_{Am1}** en présence d'un catalyseur **K1** pour obtenir un courant de produit brut **S_{RP1}** comprenant de la triacétonamine, de l'acétone,
(b) séparation par distillation au moins partielle d'acétone à partir de **S_{RP1}**, ce qui permet d'obtenir un courant gazeux **S_{Ac2}** comprenant de l'acétone,
(c) absorption au moins partielle du courant gazeux **S_{Ac2}** à contre-courant dans un courant d'acétone liquide **S_{Ac3}**, ce qui permet d'obtenir un courant d'acétone liquide **S_{Ac4},**
(d) répétition de l'étape (a), le courant d'acétone **S_{Ac4}** étant utilisé en tant que courant d'acétone **S_{Ac1}.**

2. Procédé selon la revendication 1, dans lequel la séparation par distillation au moins partielle ayant lieu dans l'étape (b) de l'acétone à partir de **S_{RP1}** a lieu dans une colonne de distillation.

3. Procédé selon la revendication 1 ou 2, dans lequel la plage de températures utilisée lors de la distillation de l'étape (b) se trouve en dessous de la température d'ébullition de la triacétonamine.

4. Procédé selon l'une des revendications 1 à 3, dans lequel, avant et/ou pendant la séparation par distillation de l'acétone à partir du courant de produit brut **S_{RP1}** selon l'étape (b), on ajoute de l'eau au courant de produit brut **S_{RP1}** et on fait réagir au moins partiellement avec l'eau au moins l'un des sous-produits du courant de produit brut **S_{RP1}** choisi dans le groupe consistant en la phorone, l'alcool diacétonique, la diacétonamine, l'acétonine, l'isophorone, de façon qu'au moins l'un des sous-produits se trouvant dans le courant de produit brut **S_{RP1}** soit au moins partiellement dissocié en acétone.

5. Procédé selon l'une des revendications 1 à 4, dans lequel l'étape (a) est mise en œuvre à une température de 20 °C à 180 °C.

6. Procédé selon l'une des revendications 1 à 5, dans lequel le rapport en moles de l'acétone utilisée dans l'étape (a) à l'ammoniac utilisé dans l'étape (a) est de 3:1 à 20:1.

7. Procédé selon l'une des revendications 1 à 6, dans lequel **K1** est un catalyseur hétérogène.

8. Procédé selon l'une des revendications 1 à 7, dans lequel dans l'étape (c), une partie de l'acétone se trouvant dans le courant gazeux **S_{Ac2}** obtenu dans l'étape (b) est absorbée dans le courant d'acétone liquide **S_{Ac3},** la partie restante étant condensée.

9. Procédé selon l'une des revendications 1 à 8, qui est mis en œuvre en continu.
